# EUROPÄISCHE PATENTSCHRIFT

(11) **EP 1 623 739 B1**
(45) Veröffentlichungstag und Bekanntmachung des Hinweises auf die Patenterteilung: **25.10.2006**
(21) Anmeldenummer: 05024826.9
(22) Anmeldetag: 06.08.2004
(51) Int. Cl.: A61N 5/10

(54) **Volumetrische Bildgebung an einem Strahlentherapiegerät**
Volumetric imaging in a radiation therapy device
Imagerie volumetrique pour appareil de radiothérapie

(43) Veröffentlichungstag der Anmeldung: 08.02.2006
(62) Teilanmeldung aus: 04018757.7
(73) Patentinhaber: BrainLAB AG, 85551 Kirchheim/Heimstetten (DE)
(72) Erfinder: Vilsmeier, Stefan, 80539 München (DE); Schlossbauer, Cornel, 80339 München (DE); Erbel, Stephan, 81677 München (DE); Labsik, Ulf, Dr., 85609 Aschheim (DE)
(74) Vertreter: Schwabe - Sandmair - Marx

(56) Entgegenhaltungen:
- EP-A- 0 562 585
- EP-A- 1 389 479
- WO-A-01/60236
- DE-A- 10 051 370
- US-A- 5 615 430
- US-B1- 6 269 143

## Beschreibung

Die Erfindung betrifft die volumetrische Bildgebung an einem Strahlentherapiegerät. Strahlentherapiegeräte, die im Weiteren auch LINACs (Linear Accelerators) genannt werden, dienen dem Grundsatz nach dazu, einem Patienten an einem vorbestimmten Körperpunkt eine Strahlendosis zu verabreichen, um beispielsweise einen Tumor zu behandeln. Es kommt bei diesen Anwendungen immer wieder vor, dass man kurz vor oder während der Behandlung nochmals Durchleuchtungsaufnahmen des Patientenkörpers machen möchte, um entweder dessen Positionierung zu überprüfen oder Veränderungen im Behandlungszielbereich festzustellen. Um dies zu bewerkstelligen, wird meist mit Röntgenbildgebung gearbeitet, und hier zeigt beispielsweise die DE 100 51 370 A1 eine Strahlentherapie- bzw. Radiochirurgieanordnung mit zwei Röntgenquellen, die oberhalb der Patientenliege angeordnet sind und nach unten auf einen Bilddetektor an der Patientenliege strahlen. Durch einen Vergleich der erhaltenen zweidimensionalen Röntgenbilder mit virtuellen, digital rekonstruierten Röntgenbildern aus vorab akquirierten CT-Aufnahmen kann die Verschiebung des Patienten aus der Sollposition heraus bestimmt und eine Neupositionierung vorgenommen werden.

Die Verwendung solcher zweidimensionaler Röntgenbilder gestattet es schon, Abweichungen der Patientenposition in Hinsicht auf die translatorischen und rotatorischen Freiheitsgrade festzustellen und zu korrigieren.

Wenn zusätzlich Verformungen innerhalb des Bestrahlungsbereichs aktuell festgestellt werden sollen, muss aber ein Volumendatensatz erstellt werden. Hierzu wird als mögliche Lösung in der WO 01/60236 ein System aus Linearbeschleuniger und Röntgenanlage vorgeschlagen, bei dem Röntgenquelle und Bildaufnehmer quer waagrecht zum LINAC-Strahlengang an der Gantry angeordnet sind. Die Röntgenanlage und die LINAC-Gantry bilden hier eine Einheit.

Es ist grundsätzlich von Nachteil, zusätzliche Apparate und damit mehr Gewicht an der LINAC-Gantry anzubringen. Die LINAC-Gantry dreht sich in vorbestimmter Weise und in vorbestimmte Stellungen, und zusätzliches Gewicht erschwert diese Drehung und macht die Einhaltung bestimmter Vorgaben im Drehvorgang schwieriger und die Bestrahlung damit ungenauer. Ein weiterer Nachteil solcher Systeme liegt darin, dass sie in nur im Ganzen als komplette Einheit bereitgestellt werden, so dass die Auf- und Umrüstung anderer Systeme nicht möglich ist, ohne vollständig auf ein solches System umzusteigen.

Es ist die Aufgabe der vorliegenden Erfindung, eine volumetrische Bildgebung an einem Strahlentherapiegerät zu ermöglichen, welche die oben genannten Nachteile des Standes der Technik überwindet. Insbesondere soll eine volumetrische Bildgebung ermöglicht werden, welche die LINAC-Bewegung nicht beeinträchtigt, und bevorzugt soll es ermöglicht werden, bestehende Systeme ohne schwerwiegende Umrüstungen für die volumetrische Bildgebung verwendbar zu machen.

Diese Aufgabe wird erfindungsgemäß durch ein Verfahren zur volumetrischen Bildgebung an einem Strahlentherapiegerät gemäß dem Anspruch 1 gelöst. Die Erfindung offenbart ferner ein Verfahren zur Patientenpositionierung an einem Strahlentherapiegerät, bei dem das vorgenannte Bildgebungsverfahren eingesetzt werden kann, sowie eine Vorrichtung zur volumetrischen Bildgebung an einem Strahlentherapiegerät gemäß dem Anspruch 9. Weiterhin umfasst die vorliegende Erfindung ein Programm, das, wenn es auf einem Computer läuft oder in einem Computer geladen ist, den Computer veranlasst, ein Verfahren durchzuführen, wie es oben beschrieben wurde, sowie ein Computerprogramm-Speichermedium, das ein solches Programm aufweist.

Gemäß einem ersten Aspekt der vorliegenden Erfindung umfasst diese ein Verfahren zur volumetrischen Bildgebung an einem Strahlentherapiegerät, bei dem
- ein Körper, von dem ein volumetrischer Bilddatensatz erstellt werden soll, auf der Couch des Strahlentherapiegeräts positioniert wird;
- die Couch oder die Liegefläche der Couch oder der Körper selbst um eine raumfeste Achse gedreht wird;
- während der Drehung mehrere Röntgenbilder des Körpers oder eines Teils davon mittels mindestens einem vom Bestrahlungsgerät separaten Strahlungsquellen/Bildaufnehmersystem erstellt und gespeichert werden, dessen Strahlungsverlauf im wesentlichen nicht parallel zu der Achse liegt;
- die jeweilige Drehstellung der Couch, der Liegefläche der Couch, oder des Köpers selbst bei der Bilderstellung erfasst und dem Bild zugeordnet wird; und bei dem
- mittels eines Computersystems durch Bildverarbeitung und -zuordnung aus den Röntgenbildern ein volumetrischer Bilddatensatz des Körpers rekonstruiert wird.

Anders ausgedrückt wird gemäß der vorliegenden Erfindung nicht mehr eine gesamte Röntgenanlage gedreht, um einen Volumendatensatz zu erhalten, sondern die hierzu erforderliche Drehbewegung wird durch eine Drehung des Patienten erzeugt. Dadurch wird vermieden, dass die gesamte Gantry-Einheit umgebaut oder ersetzt werden muss, um volumetrische Bilddaten zu erhalten, es genügt, die Patientenliege oder Couch bzw. deren Auflage drehbar zu gestalten, was sich als einfacher herausstellen wird, da solche Patientenliegen ohnehin meist Bewegungseinrichtungen aufweisen. Die LINAC-Gantry erhält erfindungsgemäß kein zusätzliches Gewicht und kann damit schon grundsätzlich genauer arbeiten und einfacher gedreht werden. Röntgenanlagen, die separat vorhanden sind, können oftmals ohne Modifikation weiterverwendet werden.

Bei einer bevorzugten Ausführungsform des erfindungsgemäßen Verfahrens verläuft die raumfeste Achse durch das Isozentrum des Strahlentherapiegeräts, insbesondere senkrecht zur Drehachse des Strahlentherapiegeräts und bevorzugt vertikal. Die Drehung der Couch oder der Liegefläche der Couch kann eine kontinuierliche Drehung insbesondere mit konstanter Geschwindigkeit sein. Die Couch wird bevorzugt um einen Winkel von mindestens 180 Grad gedreht um eine optimale Bildanzahl zu erhalten. Es hängt von den Systemparametern ab, in wie weit der Drehwinkel über 180 Grad hinaus gesteigert werden soll. Natürlich kommen bei der Verwendung geeigneter Bildverarbeitungsprogramme auch Drehwinkelbereiche in Frage, die geringer und möglicherweise weitaus geringer sind als 180 Grad.

Gemäß einer vorteilhaften Ausführungsvariante der Erfindung werden die Röntgenbilder durch zwei Strahlungsquellen / Bildaufnehmersysteme, erstellt deren Strahlengänge sich am Körper oder am Körperteil (welches das zu bestrahlende Zielgebiet enthält) kreuzen, wobei die Strahlengänge in einem Winkel zueinander stehen, der vorzugsweise etwa 90 Grad beträgt. Der volumetrische Bilddatensatz, der gemäß dem erfindungsgemäßen Verfahren rekonstruiert wird, kann ein rekonstruierter CT-Datensatz sein.

Es besteht ferner die Möglichkeit, die Drehstellung der Couch oder der Liegefläche der Couch und/oder die Position des Körpers oder des Patienten mittels eines computergestützten medizinischen Navigations- und Trackingsystems zu erfassen und zu verfolgen.

Gemäß einem weiteren Aspekt betrifft die vorliegende Erfindung ferner ein Verfahren zur Patientenpositionierung an einem Strahlentherapiegerät, bei dem
- ein Patient auf einer Couch eines Strahlentherapiegeräts positioniert und mittels eines computergestützten medizinischen Navigations- und Trackingsystems die Position des Patienten erfasst und verfolgt wird;
- mittels eines vorgenannten Verfahrens ein volumetrischer Bilddatensatz eines Bestrahlungszielgebiets erstellt, und die aktuelle Raumposition des rekonstruierten Bilddatensatzes festgestellt wird;
- mittels des Navigationssystems eine Sollposition für das Bestrahlungszielgebiet im Bereich des Einstrahlungspunktes des Strahlentherapiegeräts unter Verwendung vorab akquirierter Patienten-Volumenbilddaten, insbesondere CT- oder MR- Volumenbilddaten, festgelegt wird;
- die Abweichung zwischen der Sollposition und der aktuellen Raumposition ermittelt wird; und
- der Patient bzw. das Bestrahlungszielgebiet durch eine Verlagerung der Couch mittels einer der Abweichung entsprechenden Positionskorrektur in die Sollposition gebracht wird.

Somit lässt sich die volumetrische Bildgebung gemäß der vorliegenden Erfindung auch dazu nutzen, eine genaue Patientenpositionierung während der Bestrahlung sicherzustellen. Natürlich lassen sich die bei der volumetrischen Bildgebung erfassten Daten auch noch anderweitig nutzen; sie enthalten nämlich aktuelle Daten über Verformungen im Bestrahlungszielgebiet, die ebenfalls während der Bestrahlung Berücksichtigung finden können.

Gemäß noch einem weiteren Aspekt betrifft die vorliegende Erfindung deshalb ferner ein Verfahren zur Bildunterstützung der Arbeit mit einem Strahlentherapiegerät, bei dem
- ein Patient auf einer Couch eines Strahlentherapiegeräts positioniert und mittels eines computergestützten medizinischen Navigations- und Trackingsystems die Position des Patienten erfasst und verfolgt wird;
- mittels eines vorgenannten Verfahrens ein volumetrischer Bilddatensatz eines Bestrahlungszielgebiets erstellt, und die aktuelle Kontur des Bestrahlungsziels oder interner Organe ermittelt wird.
- die ermittelte Kontur mit der Kontur aus einem Bestrahlunsplan verglichen wird;
- die Behandlungsparameter des Strahlentherapiegeräts an die neu ermittelte Kontur des Zielgebiets angepasst wird.

Anstatt den Patienten zu verschieben, kann beispielsweise auch der Kollimator des Strahlentherapiegeräts so angepasst werden, dass das Zielgebiet richtig getroffen wird. Die Informationen über das Zielvolumen können nicht nur für eine Verschiebung verwendet werden, sondern auch, um den Plan anzupassen oder neuzurechnen. Wenn sich zum Beispiel die Prostata vergrößert hat, wir das Bestrahlungsfeld auch vergrößert.

Ferner umfasst die Erfindung noch eine Vorrichtung zur volumetrischen Bildgebung an einem Strahlentherapiegerät, mit einem Strahlentherapiegerät, dem eine Couch zugeordnet ist, auf der ein Körper, von dem ein volumetrischer Bilddatensatz erstellt werden soll, positioniert werden kann, und mit mindestens einem vom Bestrahlungsgerät separaten Strahlungsquellen/Bildaufnehmersystem, mittels dem während der Drehung der Couch mehrere Röntgenbilder des Körpers oder eines Teils davon erstellt werden können, wobei der Strahlungsverlauf des Strahlungsquellen/Bildaufnehmersystems im wesentlichen nicht auf der Achse liegt, wobei die Vorrichtung aufweist:
- eine Drehvorrichtung, mittels der die Couch oder die Liegefläche der Couch oder der Patient selbst um eine raumfeste Achse drehbar angeordnet ist;
- eine Trackingvorrichtung, welche die jeweilige Drehstellung der Couch oder der Liegefläche der Couch bei der Bilderstellung erfassen und dem Bild zuordnen kann,und
- ein Computersystem, mit dem die Drehstellung und die Bildzuordnung gespeichert werden und welches durch Bildverarbeitung und -zuordnung aus den Röntgenbildern einen volumetrischen Bilddatensatz des Körpers rekonstruiert.

Die Vorrichtung kann die oben für das Verfahren beschriebenen Merkmale vorrichtungsgemäß umsetzen, und sie erzielt dem gemäß dieselben Vorteile.

Die Erfindung wird durch die anliegenden unabhängigen Patentansprüche definiert. Die abhängigen Patentansprüche definieren vorteilhafte Ausführungsformen der Erfindung.

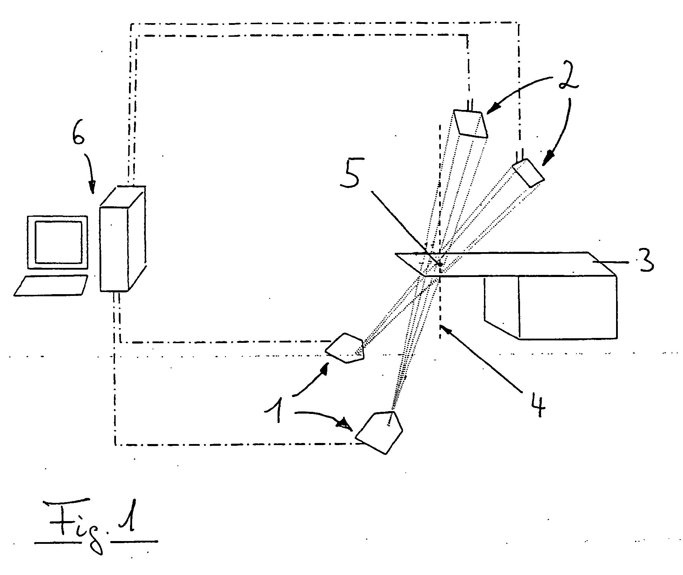

Die Erfindung, die insofern alle hierin beschriebenen Merkmale einzeln oder in jedweder Kombination umfassen kann, wird im Weiteren anhand einer Ausführungsform näher erläutert, wobei auf die einzige beiliegende Figur 1 Bezug genommen wird, welche schematisch eine Vorrichtung zeigt, mittels welcher die Erfindung umgesetzt werden kann.

In der Figur 1 ist eine Couch 3 schematisch dargestellt, die einen Couch-Grundkörper aufweist, auf dem eine Liegefläche für den Patienten angeordnet ist. Die Couch 3 steht vor einer LINAC-Gantry, die der besseren Übersichtlichkeit wegen nicht dargestellt ist. Es handelt sich um eine herkömmliche LINAC-Gantry ohne Röntgenbilderstellungs-Zusatzeinheiten.

Das Isozentrum der LINAC-Bestrahlung, also der Einstrahlungspunkt, der sich ergibt, wenn die LINAC-Gantry beim Bestrahlen gedreht wird, ist in der Zeichnung mit dem Bezugszeichen 5 angegeben. Durch das Isozentrum 5 verläuft die raumfeste, vertikale Achse 4, welche die Achse ist, um die sich zur Ausführung des erfindungsgemäßen Verfahrens die Couch 3 drehen kann. Dies kann beispielsweise dadurch bewerkstelligt werden, dass die Couch 3 auf einem Drehteller am Boden angeordnet wird. Anzumerken ist noch, dass die Achse 4 nicht unbedingt eine Isozentrums-Achse sein muss; es genügt wenn sie raumfest und ihr Verlauf koordinatenmäßig bekannt ist.

Zwei Röntgenröhren, jeweils mit dem Bezugszeichen 1 bezeichnet, sind bei der dargestellten Ausführungsform unterhalb der Gantry und des Patiententisches, im vorliegenden Fall im Boden montiert. Oberhalb der Couch 3, bevorzugt an der Decke befestigt, befinden sich zwei Röntgendetektoren, die jeweils das Bezugszeichen 2 tragen. Diese können insbesondere aus amorphen Silizium aufgebaut sein.

Ein Computersystem 6 ist mit den Röntgenröhren 1 und den Röntgendetektoren 2 verbunden. Es dient dazu, die Röntgenbilder zu akquirieren und aus den Bildinformationen der Röntgenbilder einen Volumendatensatz zu rekonstruieren, beispielsweise einen rekonstruierten CT-Datensatz.

Des Weiteren können noch einige Einrichtungen zur Verfügung gestellt werden, die in der Figur 1 nicht gezeigt sind. Beispielsweise ist es möglich, ein Navigations- oder Trackingsystem bereitzustellen, das zu seinen herkömmlichen Funktionen im Rahmen der vorliegenden Erfindung noch die Aufgabe übernehmen kann, den Drehwinkel der Couch zu messen. Natürlich kann der Drehwinkel auch direkt, beispielsweise an einem Drehteller durch bekannte Winkel-Messvorrichtungen ermittelt werden. Wenn aber, wie dies oft der Fall ist, ohnehin ein Navigationssystem bzw. ein Trackingsystem verwendet wird, um die Bestrahlung zu planen und zu unterstützen, kann dieses auch verwendet werden, um den Drehwinkel für die Couch 3 zu bestimmen.

Ferner ist es möglich, ein Kalibrierphantom mit röntgensichtbaren Markern zur Bestimmung der räumlichen Lage des Röntgensystems bereitzustellen, das vorteilhafterweise folgende Funktionen erfüllen kann: Es dient zur Definition eines räumlichen Koordinatensystems mit Ursprung im Isozentrum, wobei die vertikale (Z-)Achse des Koordinatensystems der Rotationsachse entspricht, die Y-Achse, die Null-Grad-Richtung der Drehvorrichtung definiert und senkrecht zur Z-Achse steht, und wobei die X-Achse senkrecht zur Y- und zur Z-Achse steht und ein rechtshändiges Koordinatensystem definiert (LINAC-Koordinatensystem). Es dient weiterhin dazu, die räumliche Lage der Röntgenquellen und der Detektoren bezüglich des LINAC-Koordinatensystems zu definieren, und außerdem dient es noch dazu, die Parameter der projektiven Abbildungen von Punkten aus dem LINAC-Koordinatensystem auf Punkte in den aufgenommen Röntgenbildern zu bestimmen.

Etwas allgemeiner ausgedrückt betrifft die vorliegende Erfindung in einer bevorzugten Ausführungsform ein System zur Rekonstruktion von 3D-Datensätzen aus Röntgenbildern. Dabei werden Röntgenbilder aus zwei fest installierten Röntgenquellen 1 geschossen und aus Röntgendetektoren 2 ausgelesen. Der Patient befindet sich auf einer um eine feste Achse 4 drehbaren Couch 3. Zur Rekonstruktion von 3D-Datensätzen werden vom Patienten Röntgenbilder erstellt, wobei die Couch 3 von Bild zu Bild jeweils weiter gedreht wird. Dabei steht die Drehachse 4 der Couch und damit des Patienten nicht orthogonal zur Richtung von einer Röntgenquelle 1 zum Röntgendetektor 2. Die Röntgenquellen bzw. Röntgenröhren 1 und die Röntgendetektoren 2 sind so angebracht, dass sich die Strahlen ungefähr in einem Winkel von 90 Grad schneiden. Die beiden Kombinationen aus Röntgenquelle 1 und Röntgendetektor 2 sind zueinander kalibriert. Auf diese Weise können beide Röntgenquellen 1 genutzt werden, um Bilder zu erzeugen, die zur Rekonstruktion des CT-Datensatzes verwendet werden. Dies beschleunigt einerseits die Zeit zur Aufnahme der Röntgenbilder, andererseits wird der Winkelbereich, in dem Röntgenbilder vom Patienten aufgenommen werden können, um 90 Grad vergrößert. Der Drehwinkel der Couch wird mit Hilfe eines geeigneten Systems gemessen, welches beispielsweise eine Infrarot-Trackingsystem zum Verfolgen von passiven Markern sein kann. Dazu wird ein geeigneter Referenzstern mit Infrarotmarkern an der Couch befestigt.

Aus den aufgenommenen Röntgenbildern wird mit Hilfe geeigneter, computergestützter Verfahren ein 3D-Datensatz rekonstruiert. Hierbei ist der zu rekonstruierende Bereich vorzugsweise so groß zu wählen, dass die Rückprojektion des CT-Datensatzes auf einen der beiden Röntgendetektoren 2 aus jedem möglichen Couchwinkel die gesamte Fläche des Detektors 2 abdeckt.

Im Weiteren wird nun ein etwas detaillierterer Verfahrensablauf bei der praktischen Umsetzung der vorliegenden Erfindung erläutert. Am Anfang steht die Kalibrierung, wobei das Kalibrierphantom in Isozentrumsposition 5 auf der Couch 3 bzw. auf der Liegefläche der Couch 3 platziert wird. Nunmehr werden mit Unterstützung des Computersystems 6 zwei Röntgenbilder aus den beiden Röntgenquellen 1 aufgenommen, und im Computersystem 6 werden die Projektionen aller röntgensichtbaren Marker in beiden Röntgenbildern mit Hilfe einer Bildverarbeitungssoftware automatisch detektiert. Zum Abschluss der Kalibrierung werden aus der Lage des Phantoms und der Projektion der röntgensichtbaren Marker mittels des Computersystems die benötigten Parameter errechnet, also LINAC-Koordinatensystem, räumliche Lage der Röntgenquellen und Detektoren 1, 2 und Parameter der projektiven Abbildungen.

Beim der eigentlichen Erstellung und Akquirierung der notwendigen Bildinformationen werden die folgenden Schritte durchgeführt. Der Patient wird auf der Couch 3 positioniert, und zwar so, dass der interessante Bereich sich um das Isozentrum herum befindet. Dieser Bildbereich wird der Bereich sein, in dem das Bestrahlungsziel liegt, also beispielsweise ein Tumor.

Die Couch 3 wird dann auf einen Startwinkel gedreht und der Akquisitionszyklus beginnt. Im Akquisitionszyklus findet eine konstante Rotation der Couch 3 auf ihrer Drehvorrichtung statt, und zwar um die Achse 4. Dabei werden Röntgenbilder aufgenommen, und zwar in einer solchen Weise, dass bei der Aufnahme jeweils ein bestimmter Drehwinkel einem bestimmten Zeitpunkt während einer kontinuierlichem Aufnahme oder einem einzelnen Röntgenbild zugeordnet werden kann. Die Bilddaten und die Rotationsparameter werden im Computersystem 6 gespeichert, und dieser Vorgang wird solange wiederholt, bis der Endwinkel erreicht ist.

Die weitere Arbeit wird dann von dem Computersystem 6 erledigt, das aus den aufgenommenen Röntgenbildern mit Hilfe geeigneter computergestützter Verfahren (Bildverarbeitung) einen CT-Datensatz aus den Cone-Beam-Röntgenbildern rekonstruiert.

Damit kann mit nur wenigen Modifikationen an Strahlentherapie-Vorrichtungen in einfacher Weise ein aktueller volumetrischer Bilddatensatz erhalten werden, der gemäß einer weiteren Ausführungsform auch noch dazu verwendet werden kann, den Patienten exakt zu positionieren.

## Patentansprüche

1. Verfahren zur volumetrischen Bildgebung an einem Strahlentherapiegerät, bei dem
- ein Körper, von dem ein volumetrischer Bilddatensatz erstellt werden soll, auf der Couch (3) des Strahlentherapiegeräts positioniert wird;
- die Couch oder die Liegefläche der Couch oder der Körper selbst um eine raumfeste Achse (4) gedreht wird;
- während der Drehung mehrere Röntgenbilder des Körpers oder eines Teils davon mittels mindestens einem vom Bestrahlungsgerät separaten Strahlungsquellen/Bildaufnehmersystem (1, 2) erstellt und gespeichert werden, dessen Strahlungsverlauf im wesentlichen nicht parallel zu der Achse (4) liegt;
- die jeweilige Drehstellung der Couch (3) oder der Liegefläche der Couch (3) oder unmittelbar des Körpers selbst bei der Bilderstellung erfasst und dem Bild zugeordnet wird; und bei dem
- mittels eines Computersystems (6) durch Bildverarbeitung und -zuordnung aus den Röntgenbildern ein volumetrischer Bilddatensatz des Körpers rekonstruiert wird.

2. Verfahren nach Anspruch 1, bei dem die raumfeste Achse durch das Isozentrum des Strahlentherapiegeräts verläuft, insbesondere senkrecht zur Drehachse des Strahlentherapiegeräts angeordnet ist und bevorzugt vertikal verläuft.

3. Verfahren nach Anspruch 1 oder 2, bei dem die Drehung der Couch eine kontinuierliche Drehung, insbesondere mit konstanter Geschwindigkeit ist.

4. Verfahren nach einem der Ansprüche 1 bis 3, bei dem die Couch um einen Winkel von mindestens 180° insbesondere 180° bis 190°, gedreht wird.

5. Verfahren nach einem der Ansprüche 1 bis 4, bei dem die Röntgenbilder durch zwei Strahlungsquellen/Bildaufnehmersysteme (1, 2) erstellt werden, deren Strahlengänge sich am Körper oder am Teil des Körpers kreuzen, wobei die Strahlengänge in einem Winkel zueinander stehen, der vorzugsweise etwa 90° beträgt.

6. Verfahren nach einem der Ansprüche 1 bis 5, bei dem als volumetrischer Bilddatensatz ein 3D-Datensatz rekonstruiert wird.

7. Verfahren nach einem der Ansprüche 1 bis 6, bei dem die Drehstellung der Couch (3) oder der Liegefläche der Couch (3) und/oder die Position des Körpers oder des Patienten mittels eines computergestützten medizinischen Navigations- und Trackingsystems (6) erfasst und verfolgt wird.

8. Verfahren zur Patientenpositionierung an einem Strahlentherapiegerät, bei dem
- ein Patient auf einer Couch (3) eines Strahlentherapiegeräts positioniert und mittels eines computergestützten medizinischen Navigations- und Trackingsystems (6) die Position des Patienten erfasst und verfolgt wird;
- mittels eines Verfahrens nach einem der Ansprüche 1 bis 6 ein volumetrischer Bilddatensatz eines Bestrahlungszielgebiets erstellt, und die aktuelle Raumposition des rekonstruierten Bilddatensatzes festgestellt wird;
- mittels des Navigationssystems eine Sollposition für das Bestrahlungszielgebiet im Bereich des Einstrahlungspunktes des Strahlentherapiegeräts unter Verwendung vorab akquirierter Patienten-Volumenbilddaten, insbesondere CT- oder MR- Volumenbilddaten, festgelegt wird;
- die Abweichung zwischen der Sollposition und der aktuellen Raumposition ermittelt wird; und
- der Patient bzw. das Bestrahlungszielgebiet durch eine Verlagerung der Couch (3) mittels einer der Abweichung entsprechenden Positionskorrektur in die Sollposition gebracht wird.

9. Verfahren zur Bildunterstützung der Arbeit mit einem Strahlentherapiegerät, bei dem
- ein Patient auf einer Couch eines Strahlentherapiegeräts positioniert und mittels eines computergestützten medizinischen Navigations- und Trackingsystems die Position des Patienten erfasst und verfolgt wird;
- mittels eines Verfahrens nach einem der Ansprüche 1 bis 6 ein volumetrischer Bilddatensatz eines Bestrahlungszielgebiets erstellt, und die aktuelle Kontur des Bestrahlungsziels oder interner Organe ermittelt wird.
- die ermittelte Kontur mit der Kontur aus einem Bestrahlunsplan verglichen wird;
- die Behandlungsparameter des Strahlentherapiegeräts an die neu ermittelte Kontur des Zielgebiets angepasst wird.

10. Programm, das, wenn es auf einem Computer läuft oder in einem Computer geladen ist, den Computer veranlasst, ein Verfahren gemäß einem der Ansprüche 1 bis 9 durchzuführen.

11. Computerprogramm-Speichermedium, das ein Programm nach Anspruch 10 aufweist.

## Claims

1. A method for volumetric imaging on a radiotherapy apparatus, wherein:
- a body, of which a volumetric image data set is to be produced, is positioned on the couch (3) of the radiotherapy apparatus;
- the couch or the bearing area of the couch or the body itself is rotated about a spatially fixed axis (4);
- during rotation, multiple x-ray images of the body or of a part of the body are produced and stored by means of at least one radiation source/image recorder system (1, 2) which is separate from the irradiation apparatus and whose radiation path is substantially not parallel to the axis (4);
- the rotational position of the couch (3) or the bearing area of the couch (3) or directly the body itself is detected, while the images are produced, and assigned to the image in each case; and wherein
- a volumetric image data set of the body is reconstructed from the x-ray images by image processing and assignment, by means of a computer system (6).

2. The method according to claim 1, wherein the spatially fixed axis passes through the isocentre of the radiotherapy apparatus, in particular perpendicular to the rotational axis of the radiotherapy apparatus and preferably vertically.

3. The method according to claim 1 or 2, wherein the rotation of the couch is a continuous rotation, in particular at a constant speed.

4. The method according to any one of claims 1 to 3, wherein the couch is rotated by an angle of at least 180°, in particular 180° to 190°.

5. The method according to any one of claims 1 to 4, wherein the x-ray images are produced by two radiation source/image recorder systems (1, 2) whose radiation paths cross on the body or part of the body, and the radiation paths are at an angle to each other which is preferably about 90°.

6. The method according to any one of claims 1 to 5, wherein a 3D data set is reconstructed as the volumetric image data set.

7. The method according to any one of claims 1 to 6, wherein the rotational position of the couch (3) or the bearing area of the couch (3) and/or the position of the body or patient is detected and tracked by means of a computer-assisted medical navigation and tracking system.

8. A method for patient positioning on a radiotherapy apparatus, wherein:
- a patient is positioned on a couch (3) of a radiotherapy apparatus and the position of the patient is detected and tracked by means of a computer-assisted medical navigation and tracking system (6);
- a volumetric image data set of an irradiation target region is produced by means of a method according to any one of claims 1 to 6, and the current spatial position of the reconstructed image data set is determined;
- a desired position for the irradiation target region is determined in the region of the irradiating point of the radiotherapy apparatus, by means of the navigation system, using previously acquired patient volume image data sets, in particular CT or MR volume data sets;
- the deviation between the desired position and the current spatial position is ascertained; and
- the patient and/or the irradiation target region is moved to the desired position, by means of a positional correction corresponding to the deviation, by relocating the couch (3).

9. A method for image-assisting the employment of a radiotherapy apparatus, wherein:
- a patient is positioned on a couch of a radiotherapy apparatus and the position of the patient is detected and tracked by means of a computer-assisted medical navigation and tracking system;
- a volumetric image data set of an irradiation target region is produced by means of a method according to any one of claims 1 to 6, and the current contour of the irradiation target or of internal organs is ascertained;
- the ascertained contour is compared with the contour from an irradiation plan;
- the treatment parameters of the radiotherapy apparatus are adapted to the newly ascertained contour of the target region.

10. A program which, when it is running on a computer or is loaded onto a computer, causes the computer to perform a method in accordance with any one of claims 1 to 9.

11. A computer program storage medium comprising a program according to claim 10.

## Revendications

1. Procédé d'imagerie volumétrique dans un appareil, de radiothérapie, dans lequel
- un corps dont un ensemble de données d'imagerie volumétrique doit être établi est positionné sur la couchette (3) d'un appareil de radiothérapie ;
- la couchette ou la surface de repos de la couchette ou le corps lui-même sont tournés autour d'un axe (4) fixe dans l'espace ;
- pendant la rotation, plusieurs images aux rayons X du corps ou d'une partie de celui-ci sont prises et enregistrées au moyen d'au moins un système de source de rayonnement/de prise de vues (1, 2), séparé de l'appareil d'irradiation, dont le trajet de rayonnement n'est essentiellement pas parallèle à l'axe (4) ;
- la position de rotation respective de la couchette(3) ou de la surface de repos de la couchette(3) ou directement du corps lui-même est saisie lors de la prise de vues et est associée à l'image ; et dans lequel
- au moyen d'un système informatique (6), par traitement et association d'images à partir des images aux rayons X, un ensemble de données d'image volumétrique du corps est reconstruit.

2. Procédé selon la revendication 1, dans lequel l'axe fixe dans l'espace passe par l'isocentre de l'appareil de radiothérapie, en particulier est disposé perpendiculairement à l'axe de rotation de l'appareil de radiothérapie et s'étend de préférence verticalement.

3. Procédé selon la revendication 1 ou 2, dans lequel la rotation de la couchette est une rotation continue, en particulier à vitesse constante.

4. Procédé selon l'une des revendications 1 à 3, dans lequel la couchette est tournée d'un angle d'au moins 180°, en particulier de 180° à 190°.

5. Procédé selon l'une des revendications 1 à 4, dans lequel les images aux rayons X sont réalisées par deux systèmes de source de rayonnement/de prise de vues (1, 2) dont les trajets de rayonnement se croisent sur le corps ou sur la partie du corps, les trajets de rayonnement faisant l'un par rapport à l'autre un angle qui est de préférence d'environ 90°.

6. Procédé selon l'une des revendications 1 à 5, dans lequel, comme ensemble de données d'image volumétrique, un ensemble de données 3D est reconstruit.

7. Procédé selon l'une des revendications 1 à 6, dans lequel la position de rotation de la couchette (3) ou de la surface de repos de la couchette(3) et/ou la position du corps ou du patient sont saisies et poursuivies au moyen d'un système médical de navigation et de poursuite (6) assisté par ordinateur.

8. Procédé de positionnement d'un patient sur un appareil de radiothérapie, dans lequel
- un patient est positionné sur une couchette (3) d'un appareil de radiothérapie et la position du patient est saisie et poursuivie au moyen d'un système médical de navigation et de poursuite (6) assisté par ordinateur ;
- au moyen d'un procédé selon l'une des revendications 1 à 6, un ensemble de données d'image volumétrique d'une zone cible d'irradiation est établi et la position spatiale actuelle de l'ensemble de données d'image reconstruit est déterminée ;
- au moyen du système de navigation, une position de consigne est définie pour la zone cible d'irradiation dans la région du point d'incidence de rayonnement de l'appareil de radiothérapie moyennant l'utilisation de données d'image volumique de patient précédemment acquises, en particulier de données d'image volumique par CT ou MR ;
- l'écart entre la position de consigne et la position spatiale actuelle est déterminé ; et
- le patient, respectivement la zone cible d'irradiation, est amené dans la position de consigne par un déplacement de la couchette (3) au moyen d'une correction de position correspondant à l'écart.

9. Procédé d'assistance par imagerie du travail avec un appareil de radiothérapie, dans lequel
- un patient est positionné sur une couchette (3) d'un appareil de radiothérapie et la position du patient est saisie et poursuivie au moyen d'un système médical de navigation et de poursuite assisté par ordinateur ;
- au moyen d'un procédé selon l'une des revendications 1 à 6, un ensemble de données d'image volumétrique d'une zone cible d'irradiation est établi et le contour actuel de la cible d'irradiation ou d'organes internes est déterminé.
- le contour déterminé est comparé au contour provenant d'un plan d'irradiation ;
- les paramètres de traitement de l'appareil de radiothérapie sont adaptés au contour nouvellement déterminé de la zone cible.

10. Programme qui, quand il tourne sur un ordinateur ou qu'il est chargé dans un ordinateur, permet à l'ordinateur d'exécuter un procédé selon une des revendications 1 à 9.

11. Support de données pour programme d'ordinateur qui comporte un programme selon la revendication 10.
